# EUROPEAN PATENT SPECIFICATION

(11) **EP 2 744 388 B1**
(45) Date of publication and mention of the grant of the patent: **01.04.2020**
(21) Application number: 12826308.4
(22) Date of filing: 21.08.2012
(51) Int. Cl.: A61B 1/00, G06F 3/01, G06F 3/038

(54) **WEARABLE USER INTERFACE**
TRAGBARE BENUTZERSCHNITTSTELLE
INTERFACE UTILISATEUR CORPORELLE

(30) Priority: 21.08.2011 US 201161525785 P
(43) Date of publication of application: 25.06.2014
(73) Proprietor: TransEnterix Europe Sàrl, 8070 Bertrange (LU)
(72) Inventor: SHOLEV, Mordehai, 37830 Amikam (IL); ATAROT, Gal, 44646 Kfar Saba (IL); FRIMER, Motti, 30900 Zichron Yaakov (IL)
(74) Representative: Lecomte & Partners
(86) International application number: PCT/IL2012/000312
(87) International publication number: WO 2013/027203

(56) References cited:
- EP-A1- 1 253 509
- AU-A1- 2007 234 510
- DE-A1-102006 031 735
- DE-A1-102007 022 120
- JP-A- H11 154 031
- US-A- 5 144 594
- US-A- 5 836 869
- US-A1- 2007 100 254
- US-A1- 2007 279 380
- US-A1- 2008 091 302
- US-A1- 2010 121 149
- US-A1- 2010 185 212
- US-A1- 2011 118 748

## Description

### FIELD OF THE INVENTION

The present invention generally relates to means for improving the interface between the surgeon and the operating medical assistant or between the surgeon and an endoscope system for laparoscopic surgery. Moreover, this present invention discloses a wearable interface for enhancing the control of an endoscope system during laparoscopic surgery.

### BACKGROUND OF THE INVENTION

In laparoscopic surgery, the surgeon performs the operation through small holes using long instruments and observing the internal anatomy with an endoscope camera. The endoscope is conventionally held by a human camera assistant (i.e. operating medical assistant) since the surgeon must perform the operation using both hands. The surgeon's performance is largely dependent on the camera's position relative to the instruments and on a stable image shown in the monitor. The main problem is that it is difficult for the operating medical assistant to hold the endoscope steady, keeping the scene upright.

Laparoscopic surgery is becoming increasingly popular with patients because the scars are smaller and their period of recovery is shorter. Laparoscopic surgery requires special training for the surgeon or gynecologist and the theatre nursing staff. The equipment is often expensive and is not available in all hospitals.

During laparoscopic surgery it is often required to shift the spatial placement of the endoscope in order to present the surgeon with an optimal view. Conventional laparoscopic surgery makes use of either human assistants who manually shift the instrumentation or, alternatively, robotic automated assistants. Automated assistants utilize interfaces that enable the surgeon to direct the mechanical movement of the assistant, achieving a shift in the camera view.

US patent 6,714,841 discloses an automated camera endoscope in which the surgeon is fitted with a head mounted light source that transmits the head movements to a sensor, forming an interface that converts the movements to directions for the mechanical movement of the automated assistant. Alternative automated assistants incorporate a voice operated interface, a directional key interface, or other navigational interfaces. The above interfaces share the following drawbacks:
a. A single directional interface that provides limited feedback to the surgeon
b. A cumbersome serial operation for starting and stopping movement directions that requires the surgeon's constant attention, preventing the surgeon from keeping the flow of the surgical procedure.

Research has suggested that these systems divert the surgeon's focus from the major task at hand. Therefore, technologies assisted by magnets and image processing have been developed to simplify interfacing control. However, these improved technologies still fail to address another complicating interface aspect of laparoscopic surgery, in that they do not allow the surgeon to signal to automated assistants, to human assistants or to surgical colleagues which instrument his attention is focused on.

Hence, there is still a long felt need for improving the interface between the surgeon and an endoscope system, surgical colleagues or human assistants for laparoscopic surgery.

US patent application published US 2008/0091302 A1 relates to an interface laparoscopy device in accordance with the preamble of appended claim 1.

EP patent application published EP 1 253 509 A1 relates to an interface laparoscopy device in accordance with the preamble of appended claim 1.

DE patent application published DE 10 2007 022 120 A1 relates to a gripping operating device for surgical applications.

### SUMMARY OF THE INVENTION

The main object of the present invention is to provide an enhanced interface laparoscopy device in accordance with appended independent claim 1 and appended dependent claims 2-4.

It is one object of the present invention to provide an enhanced interface laparoscopy device, comprising:
a. at least one endoscope, mechanically interconnected to said automated assistant; said automated assistant is adapted to maneuver said endoscope to a desired location;
b. at least one instrument;
c. at least one wearable operator comprising at least one wireless transmitter, adapted to transmit a signal once said at least one wearable operator is activated; said at least one wearable operator is in communication with said- at least one of said instrument;
d. at least one wireless receiver; adapted to receive said signal sent by said transmitter;
e. at least one laparoscopy computerized system, in communication with said wireless receiver, adapted to provide a visual onscreen depiction of said at least one instrument to be selected following the activation of said at least one wearable operator; and,
f. at least one video screen;
wherein said device is adapted to control and to direct said endoscope via said laparoscopy computerized system and said automated assistant on said instrument to be selected following the activation of said at least one wearable operator; wherein said at least one operator is wearable, and wherein said wearable operator comprises a body having at least two portions partially overlapping each other; said two portions are adapted to grasp and hold either said instrument there-between, such that a tight-fit coupling between said two portions and said instrument is obtained,
wherein one of the two portions is provided with a unidirectional or a two-way direction catch, such that the one of the two portions is adapted to be linearly moved relative to the other of the two portions.

It is another object of the present invention to provide the device as defined above, wherein said communication between said at least one wearable operators and said instrument is either wire or wirelessly coupl ing.

It is another object of the present invention to provide the device as defined above, wherein said device is adapted to control and to direct said endoscope via said laparoscopy computerized system and said automated assistant on said instrument to which said activated wearable operator is coupled.

It is another object of the present invention to provide the device as defined above, wherein said wearable operator is worn by said surgeon on a predetermined body part, wherein said predetermined body part is selected from a group consisting of the hand of said surgeon, at least one of the fingers of said surgeon, and any combination thereof; further wherein the shape of said wearable operator is selected from a group consisting of a ring, a bracelet and any combination thereof.

It is another object of the present invention to provide the device as defined above, wherein said wearable operator is coupled to a predetermined location on said instrument by means of an adaptor.

It is another object of the present invention to provide the device as defined above, wherein said wearable operator is adjustable so as to fit said predetermined location of said different instruments, each of which is characterized by a different size and shape.

It is another object of the present invention to provide the device as defined above, wherein said wearable operator comprises a body having two portions partially overlapping each other; said two portions are adapted to grasp and hold either said instrument there-between, such that a tight-fit coupling between said two portions and said instrument is obtained.

It is another object of the present invention to provide the device as defined above, wherein one of said two portions is rotationally movable relative to the other, such that when said wearable operator is coupled to said instrument, fine-tuned movement

It is another object of the present invention to provide the device as defined above, wherein said wireless transmitter is freestanding.

It is another object of the present invention to provide the device as defined above, wherein each of said at least one instrument is fitted with at least one of said wireless transmitters.

It is another object of the present invention to provide the device as defined above, wherein said wireless transmitter is adapted to locate the position of at least one of said instruments.

It is another object of the present invention to provide the device as defined above, wherein a selection of said at least one instrument is obtained by clicking on said at least one wearable operator.

It is another object of the present invention to provide the device as defined above, wherein the activation of said at least one wearable operator is obtained by depression on the same, voice activating the same, prolonged depression on the same, double clicking on the same and any combination thereof.

It is another object of the present invention to provide the device as defined above, wherein said laparoscopy computerized system directs said endoscope by using image information shown on said video screen without said help of assistants.

It is another object of the present invention to provide the device as defined above, wherein said conventional laparoscopy computerized system comprises at least one surgical instrument spatial location software, adapted to locate the 3D spatial position of said at least one instrument further wherein said conventional laparoscopy computerized system comprises at least one automated assistant maneuvering system; said automated assistant maneuvering system is coupled to said endoscope and is adapted to direct said endoscope to said at least one instrument, said instrument selected following the activation of said at least one wearable operator.

It is another object of the present invention to provide the device as defined above, wherein each transmitted signal from said wearable operator and said wireless transmitter is matched to at least one of said instruments.

### DETAILED DESCRIPTION OF THE EMBODIMENTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide means for improving the interface between the surgeon and an endoscope system for laparoscopic surgery.

The present invention provides a wearable user interface operator (referred to also as the 'wearable operator').

According to one embodiment, the wearable user interface is attached to the operating tool.

According to another embodiment, the interface is linked/attached to a predetermined body part of the surgeon. Said body part is selected from a group consisting of: the hand of the surgeon, at least one of the fingers of the surgeon, the thigh of the surgeon, the neck of the surgeon, at least one of the legs of the surgeon, the knee of the surgeon, the head of the surgeon and any combination thereof.

The present invention can be also utilized to improve the interface between the surgeon and the operating medical assistant and/or the surgeon's colleagues. Moreover, the present invention can be also utilized to control and/or direct an automated endoscope assistant to focus the endoscope on the desired instrument of the surgeon via output from the wearable operator, said output controlled by the surgeon. Furthermore, the device is adapted to direct the operating medical assistant to focus on the desired instrument of the surgeon.

According to another embodiment of the present disclosure, a wearable operator is provided. The wearable operator comprising:
a. at least two portions at least partially overlapping each other; said two portion are adapted to rotate and tilt relative to each other;
b. at least one wireless transmitter, adapted to transmit a signal once said at least one wearable operator is activated.

It is another object of the present disclosure to provide the system as defined above, wherein said at least one wireless transmitter is attached to said at least one laparoscopic instrument.

It is another object of the present disclosure to provide the system as defined above, wherein said identifying to said user of said laparoscopic instrument is effected via a visual depiction of said laparoscopic instrument on a display.

It is another object of the present disclosure to provide the system as defined above, further comprising an automated assistant for controlling an endoscopic camera.

It is another object of the present disclosure to provide the system as defined above, wherein said computerized platform tracks said laparoscopic instrument using image information received from said endoscopic camera.

It is another object of the present disclosure to provide the system as defined above, wherein said computerized platform controls said automated assistant.

It is another object of the present disclosure to provide the system as defined above, wherein said computerized platform visually identifies said laparoscopic instrument to said user upon activation of said transmitter.

It is another object of the present disclosure to provide the system as defined above, wherein at least one of said wearable operators is either wire or wirelessly coupled to said at least one of said laparoscopic instruments.

It is another object of the present disclosure to provide the system as defined above, wherein said computerized platform is adapted to track and to identify said laparoscopic instrument to which said wearable operator is coupled.

It is another object of the present disclosure to provide the system as defined above, wherein said wearable operator is worn by said surgeon on a predetermined body part.

It is another object of the present disclosure to provide the system as defined above, wherein said predetermined body part is selected from a group consisting of the hand of said surgeon, at least one of the fingers of said surgeon, the thigh of said surgeon, the neck of said surgeon, at least one of the legs of said surgeon, the knee of said surgeon, the head of said surgeon and any combination thereof.

It is another object of the present disclosure to provide the system as defined above, wherein the shape of said wearable operator is selected from a group consisting of a ring, a bracelet and any combination thereof.

It is another object of the present disclosure to provide the system as defined above, wherein said wearable operator is coupled to a predetermined location on said instrument by means of an adaptor.

It is another object of the present disclosure to provide the system as defined above, wherein said wearable operator is adjustable so as to fit said predetermined location of said different instruments, each of which is characterized by a different size and shape.

It is another object of the present disclosure to provide the system as defined above, wherein said wearable operator comprises a body having at least two portions at least partially overlapping each other; said two portions are adapted to grasp and hold either said instrument or said predetermined body part there-between, such that a tight-fit coupling between said two portions and said instrument or said predetermined body part is obtained.

It is another object of the present disclosure to provide the system as defined above, wherein one of said two portions is rotationally movable relative to the other, such that when said wearable operator is coupled to said instrument, fine-tuned movement of said two body portions is obtainable so as to provide said tight-fit coupling between said two portions and said instrument or said predetermined body part.

It is another object of the present disclosure to provide the system as defined above, wherein said two portions are rotationally movable relative to each other, such that when said wearable operator is coupled to said instrument, fine-tuned movement of said two body portions is obtainable so as to provide said tight-fit coupling between said two portions and said instrument or said predetermined body part.

It is another object of the present disclosure to provide the system as defined above, wherein said wearable operator comprises (a) at least one flexible and stretchable strip; and, (b) loop-closing means adapted to close a loop with said at least one flexible and stretchable strip; said at least one flexible and stretchable strip and said loop-closing means are provided so as to fit said wearable operator to at least one selected from a group consisting of (a) said predetermined location of said different instruments; (b) said predetermined body part of said surgeon, each of which is characterized by a different size and shape.

It is another object of the present disclosure to provide the system as defined above, wherein said flexible and stretchable strip is made of material selected from a group consisting of silicone, rubber and any combination thereof.

It is another object of the present disclosure to provide the system as defined above, wherein said loop-closing means is at least one unidirectional catch through which said flexible and stretchable strip is passed so as to provide a loop.

It is another object of the present disclosure to provide the system as defined above, wherein said loop-closing means is at least one peg around which said flexible and stretchable strip is passed so as to provide a loop.

It is another object of the present disclosure to provide the system as defined above, wherein said flexible and stretchable strip is characterized by a varied width along its length.

It is another object of the present disclosure to provide the system as defined above, wherein said flexible and stretchable strip is characterized by different surface roughnesses along its length.

It is another object of the present disclosure to provide the system as defined above, wherein said wireless transmitter is freestanding.

It is another object of the present disclosure to provide the system as defined above, wherein each of said at least one laparoscopic instruments is fitted with at least one of said wireless transmitters.

It is another object of the present disclosure to provide the system as defined above, wherein said wireless transmitter is adapted to locate the position of at least one of said laparoscopic instruments.

It is another object of the present disclosure to provide the system as defined above, wherein selection of said at least one laparoscopic instrument is confirmed by activating said at least one wearable operator

It is another object of the present disclosure to provide the system as defined above, wherein the activation of said at least one wearable operator is obtained by depression on the same, voice activating the same, prolonged depression on the same, double clicking on the same and any combination thereof.

It is another object of the present disclosure to provide the system as defined above, wherein said computerized platform directs an endoscope to said laparoscopic instrument by using image information shown on a video screen without said help of assistants.

It is another object of the present disclosure to provide the system as defined above, wherein each transmitted signal from said wearable operator and said wireless transmitter is matched to at least one of said instruments.

It is another object of the present disclosure to provide a method useful for the interface between a surgeon and an automated assistant; said method comprising the step of:
a. obtaining a device comprising:
   i. at least one desired laparoscopic instrument;
   ii. at least one endoscope, mechanically interconnected to said automated assistant;
   iii. at least one wearable operator comprising at least one wireless transmitter;
   iv. at least one wireless receiver;
   v. at least one laparoscopy computerized system loaded with (i) surgical instrument spatial location software; (ii) automated assistant maneuvering software; (iii) and, a software that enables a visual onscreen depiction response to the activation of said at least one wearable operator; and,
   vi. at least one video screen;
b. activating said wearable operator; thereby selecting a desired laparoscopic instrument and emitting a signal;
c. receiving said signal by said receiver;
d. maneuvering said endoscope so as to focus said endoscope on said desired laparoscopic instrument of said surgeon; and,
e. displaying said desired instrument on a screen; wherein said device is adapted to control and to direct said endoscope via said laparoscopy computerized system and said automated assistant on said instrument to be selected following the activation of said at least one wearable operator.

It is another object of the present disclosure to provide the method as defined above, additionally comprising the step of manually or automatically activating said wearable operator.

It is another object of the present disclosure to provide the method as defined above, wherein said wireless transmitter is freestanding.

It is another object of the present disclosure to provide the method as defined above, additionally comprising the step of attaching said at least one wireless transmitter to said at least one desired laparoscopic instrument.

It is another object of the present disclosure to provide the method as defined above, additionally comprising the step of identifying to said user of said desired laparoscopic instrument; further wherein said step is effected via a visual depiction of said laparoscopic instrument on a display.

It is another object of the present disclosure to provide the method as defined above, wherein said laparoscopy computerized system tracks said laparoscopic instrument using image information received from said endoscopic camera.

It is another object of the present disclosure to provide the method as defined above, wherein said laparoscopy computerized system controls said automated assistant.

It is another object of the present disclosure to provide the method as defined above, wherein said laparoscopy computerized system visually identifies said laparoscopic instrument to said user upon activation of said transmitter.

It is another object of the present disclosure to provide the method as defined above, additionally comprising step of confirming the selection of said desired instrument. It is another object of the present disclosure to provide the method as defined above, wherein said step of selecting said desired laparoscopic instrument additionally comprises the steps of (a) activating wearable operator; (b) transmitting a generic code to said receiver; (c) communicating said signal to a computer, thereby operating said automated assistant.

It is another object of the present disclosure to provide the method as defined above, wherein said step of selecting said desired laparoscopic instrument additionally comprises the step of confirming the selection of said desired laparoscopic instrument by clicking on said wearable operator.

It is another object of the present disclosure to provide the method as defined above, wherein said step of selecting said desired laparoscopic instrument additionally comprises the step confirming the selection of said laparoscopic desired instrument by a prolonged depression on said wearable operator.

It is another object of the present disclosure to provide the method as defined above, additionally comprising the step of identifying each of said desired laparoscopic instrument to said computerized system.

It is another object of the present disclosure to provide the method as defined above, additionally comprising the step of attaching said wearable operator to said laparoscopic instrument.

It is another object of the present disclosure to provide the method as defined above, additionally comprising the step of matching each transmitted code from said wearable operator and said wireless transmitter to at least one of said laparoscopic instruments.

It is another object of the present disclosure to provide the method as defined above, additionally comprising step of either wire or wirelessly coupling at least one of said wearable operators to said at least one of said instruments.

It is another object of the present disclosure to provide the method as defined above, additionally comprising step of controlling and directing said endoscope via said laparoscopy computerized system and said automated assistant on said desired laparoscopic instrument to which said activated wearable operator is coupled.

It is another object of the present disclosure to provide the method as defined above, additionally comprising step of wearing said wearable operator by said surgeon on a predetermined body part.

It is another object of the present disclosure to provide the method as defined above, additionally comprising step of selecting said predetermined body part from a group consisting of the hand of said surgeon, at least one of the fingers of said surgeon, the thigh of said surgeon, the neck of said surgeon, at least one of the legs of said surgeon, the knee of said surgeon, the head of said surgeon and any combination thereof.

It is another object of the present disclosure to provide the method as defined above, additionally comprising step of selecting the shape of said wearable operator from a group consisting of a ring, a bracelet and any combination thereof.

It is another object of the present disclosure to provide the method as defined above, additionally comprising step of coupling said wearable operator to a predetermined location on said instrument by means of an adaptor.

It is another object of the present disclosure to provide the method as defined above, additionally comprising step of adjusting said wearable operator so as to fit said predetermined location of said different instruments, each of which is characterized by a different size and shape.

It is another object of the present disclosure to provide the method as defined above, additionally comprising step of providing said wearable operator with a body having at least two portions at least partially overlapping each other; said two portions are adapted to grasp and hold either said instrument or said predetermined body part there-between, such that a tight-fit coupling between said two portions and said instrument or said predetermined body part is obtained.

It is another object of the present disclosure to provide the method as defined above, wherein one of said two portions is rotationally movable relative to the other, such that when said wearable operator is coupled to said instrument, fine-tuned movement of said two body portions is obtainable so as to provide said tight-fit coupling between said two portions and said instrument or said predetermined body part.

It is another object of the present disclosure to provide the method as defined above, additionally comprising step of coupling wherein said two portions are rotationally movable relative to each other, such that when said wearable operator is coupled to said instrument, fine-tuned movement of said two body portions is obtainable so as to provide said tight-fit coupling between said two portions and said instrument or said predetermined body part.

It is another object of the present disclosure to provide the method as defined above, wherein said wearable operator comprises (a) at least one flexible and stretchable strip; and, (b) loop-closing means adapted to close a loop with said at least one flexible and stretchable strip; said at least one flexible and stretchable strip and said loop-closing means are provided so as to fit said wearable operator to at least one selected from a group consisting of (a) said predetermined location of said different instruments; (b) said predetermined body part of said surgeon, each of which is characterized by a different size and shape.

It is another object of the present disclosure to provide the method as defined above, additionally comprising step of providing said flexible and stretchable strip to be made of material selected from a group consisting of silicone, rubber and any combination thereof.

It is another object of the present disclosure to provide the method as defined above, wherein said loop-closing means is at least one unidirectional catch through which said flexible and stretchable strip is passed so as to provide a loop.

It is another object of the present disclosure to provide the method as defined above, wherein said loop-closing means is at least one peg around which said flexible and stretchable strip is passed so as to provide a loop.

It is another object of the present disclosure to provide the method as defined above, wherein said flexible and stretchable strip is characterized by a varied width along its length.

It is another object of the present disclosure to provide the method as defined above, wherein said flexible and stretchable strip is characterized by different surface roughnesses along its length.

It is another object of the present disclosure to provide the method as defined above, wherein said wireless transmitter is freestanding.

It is another object of the present disclosure to provide the method as defined above, wherein each of said at least one instrument is fitted with at least one of said wireless transmitters.

It is another object of the present disclosure to provide the method as defined above, wherein said wireless transmitter is adapted to locate the position of at least one of said instruments.

It is another object of the present disclosure to provide the method as defined above, additionally comprising step of selecting said at least one instrument by activating said at least one wearable operator.

It is another object of the present disclosure to provide the method as defined above, additionally comprising step of activating said at least one wearable operator by depression on the same, voice activating the same, prolonged depression on the same, double clicking on the same and any combination thereof.

It is another object of the present disclosure to provide a method useful for the interface between a surgeon and an automated assistant; said method comprising the step of:
a. obtaining a surgical system comprising:
   i. at least one laparoscopic instrument;
   ii. at least one wearable operator comprising at least one wireless transmitter capable of being activated to transmit a signal;
   iii. at least one a computerized platform configured for tracking said at least one laparoscopic instrument and being capable of receiving said signal and identifying to a user a laparoscopic instrument selected by activation of said transmitter from said at least one laparoscopic instrument; wherein said wearable operator is being worn by the surgeon;
b. activating said wearable operator; thereby selecting a desired laparoscopic instrument and emitting a signal;
c. receiving said signal by said receiver;
d. maneuvering an endoscope so as to focus the same on said desired laparoscopic instrument of said surgeon; and,
e. displaying said desired instrument on a screen; wherein said system is adapted to control and to direct said endoscope via said laparoscopy computerized system and said automated assistant on said instrument to be selected following the activation of said at least one wearable operator.

It is another object of the present disclosure to provide the method as defined above, additionally comprising step of manually or automatically activating said wearable operator.

It is another object of the present disclosure to provide the method as defined above, additionally comprising step of tracking said laparoscopic instrument selected upon activation of said transmitter by means of said computerized platform.

It is another object of the present disclosure to provide the method as defined above, wherein said wireless transmitter is freestanding.

It is another object of the present disclosure to provide the method as defined above, additionally comprising step of attaching said at least one wireless transmitter to said at least one laparoscopic instrument.

It is another object of the present disclosure to provide the method as defined above, additionally comprising step of identifying to said user of said laparoscopic instrument via a visual depiction of said laparoscopic instrument on a display.

It is another object of the present disclosure to provide the method as defined above, additionally comprising step of providing an automated assistant for controlling an endoscopic camera.

It is another object of the present disclosure to provide the method as defined above, wherein said computerized platform tracks said laparoscopic instrument using image information received from said endoscopic camera.

It is another object of the present disclosure to provide the method as defined above, additionally comprising step of controlling said automated assistant by means of said computerized platform.

It is another object of the present disclosure to provide the method as defined above, wherein said computerized platform visually identifies said laparoscopic instrument to said user upon activation of said transmitter.

It is another object of the present disclosure to provide the method as defined above, wherein at least one of said wearable operators is either wire or wirelessly coupled to said at least one of said laparoscopic instruments.

It is another object of the present disclosure to provide the method as defined above, wherein said computerized platform is adapted to track and to identify said laparoscopic instrument to which said wearable operator is coupled.

It is another object of the present disclosure to provide the method as defined above, additionally comprising step of wearing said wearable operator by said surgeon on a predetermined body part.

It is another object of the present disclosure to provide the method as defined above, wherein said predetermined body part is selected from a group consisting of the hand of said surgeon, at least one of the fingers of said surgeon, the thigh of said surgeon, the neck of said surgeon, at least one of the legs of said surgeon, the knee of said surgeon, the head of said surgeon and any combination thereof.

It is another object of the present disclosure to provide the method as defined above, wherein the shape of said wearable operator is selected from a group consisting of a ring, a bracelet and any combination thereof.

It is another object of the present disclosure to provide the method as defined above, additionally comprising step of coupling said wearable operator to a predetermined location on said instrument by means of an adaptor.

It is another object of the present disclosure to provide the method as defined above, wherein said wearable operator is adjustable so as to fit said predetermined location of said different instruments, each of which is characterized by a different size and shape.

It is another object of the present disclosure to provide the method as defined above, additionally comprising step of providing said wearable operator with a body having at least two portions at least partially overlapping each other; said two portions are adapted to grasp and hold either said instrument or said predetermined body part there-between, such that a tight-fit coupling between said two portions and said instrument or said predetermined body part is obtained.

It is another object of the present disclosure to provide the method as defined above, wherein one of said two portions is rotationally movable relative to the other, such that when said wearable operator is coupled to said instrument, fine-tuned movement of said two body portions is obtainable so as to provide said tight-fit coupling between said two portions and said instrument or said predetermined body part.

It is another object of the present disclosure to provide the method as defined above, wherein said two portions are rotationally movable relative to each other, such that when said wearable operator is coupled to said instrument, fine-tuned movement of said two body portions is obtainable so as to provide said tight-fit coupling between said two portions and said instrument or said predetermined body part.

It is another object of the present disclosure to provide the method as defined above, wherein said wearable operator comprises (a) at least one flexible and stretchable strip; and, (b) loop-closing means adapted to close a loop with said at least one flexible and stretchable strip; said at least one flexible and stretchable strip and said loop-closing means are provided so as to fit said wearable operator to at least one selected from a group consisting of (a) said predetermined location of said different instruments; (b) said predetermined body part of said surgeon, each of which is characterized by a different size and shape.

It is another object of the present disclosure to provide the method as defined above, wherein said flexible and stretchable strip is made of material selected from a group consisting of silicone, rubber and any combination thereof.

It is another object of the present disclosure to provide the method as defined above, wherein said loop-closing means is at least one unidirectional catch through which said flexible and stretchable strip is passed so as to provide a loop.

It is another object of the present disclosure to provide the method as defined above, wherein said loop-closing means is at least one peg around which said flexible and stretchable strip is passed so as to provide a loop.

It is another object of the present disclosure to provide the method as defined above, wherein said flexible and stretchable strip is characterized by a varied width along its length.

It is another object of the present disclosure to provide the method as defined above, wherein said flexible and stretchable strip is characterized by different surface roughnesses along its length.

It is another object of the present disclosure to provide the method as defined above, additionally comprising step of fitting each of said at least one laparoscopic instruments with at least one of said wireless transmitters.

It is another object of the present disclosure to provide the method as defined above, wherein said wireless transmitter is adapted to locate the position of at least one of said laparoscopic instruments.

It is another object of the present disclosure to provide the method as defined above, additionally comprising step of confirming a selection of said at least one laparoscopic instrument by clicking on said at least one wearable operator It is another object of the present disclosure to provide the method as defined above, additionally comprising step of activating said at least one wearable operator by depression on the same, voice activating the same, prolonged depression on the same, double clicking on the same and any combination thereof.

It is another object of the present disclosure to provide the method as defined above, additionally comprising step of directing an endoscope to said laparoscopic instrument by using image information shown on a video screen by means of said computerized platform configured without said help of assistants.

It is another object of the present disclosure to provide a wearable operator, comprising:
(a) at least two portions at least partially overlapping each other; said two portion are adapted to rotate and tilt relative to each other;
(b) at least one wireless transmitter, adapted to transmit a signal once said at least one wearable operator is activated.

It is another object of the present disclosure to provide the wearable operator as defined above, wherein said wearable operator is worn by a user on a predetermined body part, such that activation of said wearable operator results in activation of an external instrument.

It is another object of the present disclosure to provide the wearable operator as defined above, wherein said predetermined body part is selected from a group consisting of: the hand of said surgeon, at least one of the fingers of said user, the thigh of said user, the neck of said user, at least one of the legs of said user, the knee of said user, the head of said user and any combination thereof.

It is another object of the present disclosure to provide the wearable operator as defined above, wherein said wearable operator is coupled to a predetermined location on an instrument by means of an adaptor, such that activation of said wearable operator results in activation of said instrument.

It is another object of the present disclosure to provide the wearable operator as defined above, wherein said coupling between said at least one of said wearable operators and said instrument is either wire or wirelessly coupling.

It is another object of the present disclosure to provide the wearable operator as defined above, wherein said wearable operator comprises (a) at least one flexible and stretchable strip; and, (b) loop-closing means adapted to close a loop with said at least one flexible and stretchable strip; said at least one flexible and stretchable strip and said loop-closing means are provided so as to fit said wearable operator to at least one selected from a group consisting of (a) said predetermined location of said different instruments; (b) said predetermined body part of said user, each of which is characterized by a different size and shape.

It is another object of the present disclosure to provide the wearable operator as defined above, wherein the shape of said wearable operator is selected from a group consisting of a ring, a bracelet and any combination thereof.

### BRIEF DESCRIPTION OF THE FIGURES

In order to understand the invention and to see how it may be implemented in practice, and by way of non-limiting example only, with reference to the accompanying drawings, in which
FIGs. 1a-1c is a general schematic view of an enhanced interface laparoscopic system that relies on a single wireless code signal to indicate the instrument on which to focus the endoscope, constructed in accordance with the principles of the present disclosure in a preferred embodiment thereof;
FIG. 2 is a general schematic view of an enhanced interface laparoscopic system that relies on at least two wireless signals to indicate the instrument on which to focus the endoscope;
FIG. 3 is a schematic view of the method in which the single wireless code signal choice instrumentation focus is represented on the viewing apparatus;
FIG. 4 is a schematic view of the method in which multiple wireless code signal choice of instrumentation is operated;
FIG. 5a-5e illustrates another preferred embodiment of the present disclosure;
FIG. 6 illustrates the adjustability of the wearable operator;
FIG. 7a-7d illustrates the embodiment of the wearable operator 700 of the invention and the adjustment means of the same to a surgical tool;
FIG. 8a-8c, illustrates another embodiment of the present disclosure, which provides best adjustment of the wearable operator to the operator's hand; and
FIG. 9a-9b illustrates the 'adjustability' of the wearable operator.

### DETAILED DESCRIPTION OF THE EMBODIMENTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide means and methods for improving the interface between the surgeon and an endoscope system for laparoscopic surgery.

The present invention provides a wearable user interface operator (referred to also as the 'wearable operator') as defined by claim 1. All other embodiments are for disclosure only.

According to one embodiment, the wearable user interface is attached to the operating tool.

According to another embodiment, the interface is linked/attached to a predetermined body part of the surgeon. Said body part is selected from a group consisting of: the hand of the surgeon, at least one of the fingers of the surgeon, the thigh of the surgeon, the neck of the surgeon, at least one of the legs of the surgeon, the knee of the surgeon, the head of the surgeon and any combination thereof.

The present invention can be also utilized to improve the interface between the surgeon and the operating medical assistant and/or the surgeon's colleagues. Moreover, the present invention can be also utilized to control and/or direct an automated endoscope assistant to focus the endoscope on the desired instrument of the surgeon via output from the wearable operator, said output controlled by the surgeon. Furthermore, the device is adapted to direct the operating medical assistant to focus on the desired instrument of the surgeon.

The term **"conventional laparoscopy computerized system"** refers herein to a system or/software conventionally used in the market such as Lapman, Endo Assist or AESOP.

The term **"tight-fit"** refers herein to a fit between two parts, such that said two parts are considered as coupled together.

According to a preferred embodiment of the present invention, a single wireless emission code is utilized and choice is achieved by a visible graphic representation upon a viewing screen.

According to another preferred embodiment, each instrument is fitted with a unique code wireless transmitter, and selection is achieved by depressing a control on the wearable operator.

The present invention also discloses a device joined with conventional camera assisted laparoscopic surgery systems comprising at least one wireless transmitter that may or may not be attached to the maneuvering control end of surgical instruments. Upon activation (e.g., depression) of a control on the wearable operator, either a generic or a unique code is transmitted to a receiving device connected to a computer that presents (e.g. displays) the selected surgical tool on a connected video screen. After confirmation of the selection by the depression of at least one button in the wearable operator's wireless transmitter, a code is transmitted to the receiver connected to the computer that instructs the automated surgical assistant to move the endoscope, achieving a view on the screen that is focused on the selected instrument area.

It would thus be desirable to achieve a device that allows the surgeon to identify to the laparoscopic computing system as well as to surgical colleagues to which surgical instrument attention is to be directed. By identifying the surgical instrument via the laparoscopic computing system, the endoscope directs the view to the selected focus of attention.

Therefore, in accordance with a preferred embodiment of the present invention, an enhanced interface laparoscopy device is provided. The device comprises:
a. at least one endoscope, mechanically interconnected to said automated assistant; said automated assistant is adapted to maneuver said endoscope to a desired location;
b. at least one instrument;
c. at least one wearable operator comprising at least one wireless transmitter, adapted to transmit a signal once said at least one wearable operator is activated; said at least one wearable operator is either wire or wirelessly in communication with saidat least one of said instrument;
d. at least one wireless receiver adapted to receive said signal sent by said transmitter;
e. at least one laparoscopy computerized system, in communication with said wireless receiver, adapted to provide a visual onscreen depiction of said at least one instrument to be selected following the activation of said at least one wearable operator; and,
f. at least one video screen
wherein said device is adapted to control and to direct said endoscope via said laparoscopy computerized system and said automated assistant on said instrument, said instrument to be selected following the activation of said at least one wearable operator.

In a preferred embodiment of the enhanced interface laparoscopy device, the wireless transmitter or transmitters are either freestanding or are attached to the maneuvering end of the surgical instruments. They emit the same single code so that, upon the activation (e.g., depression) of the wearable operator, they emit a signal to the receiver. The receiver communicates with a connected computer that displays a graphic symbol upon one of one of the surgical instruments depicted on the screen by the computer. On initial activation, the graphical symbol can be displayed on a randomly-chosen surgical instrument, or it can be displayed on a predefined surgical instrument.

If needed, the surgeon repeats the activation (e.g., depression) of the wearable operator resulting in a shift in the displayed graphic designator from one onscreen depiction of a surgical instrument to another until the desired instrument is reached and thereby selected. Subsequently the computer directs the automated assistant to focus the endoscope on the desired instrument area.

In a further preferred embodiment the selection of the instrument requires confirmation by varying the form of activating said wearable operator, such as a prolonged depression, double clicking or voice activation. Only upon confirmation is the computer authorized to instruct the automated assistant to focus the endoscope on the desired instrument area.

In another preferred embodiment of the invention each relevant surgical instrument is fitted at its maneuvering control end with a wireless transmitter that transmits a unique code.

In the initial stage of the procedure, the surgeon identifies each of the instruments to the computerized system by activating the wearable operator (e.g., depressing at least one key on the same) on each of the wireless transmitters fitted to the surgical instruments and matching their characteristics with a prepared database, thereby forming within the computerized system a unique signature for each of the transmitters.

Thereon, upon depression of the wearable operator attached to each surgical instrument/or on the surgeon's hand, the receiver receives the unique code, and communicates it to the computer. The computer identifies it with the preprogrammed signature and instructs the automated assistant to move the endoscope so as to achieve the desired focus.

It should be pointed out that the wearable operator can be coupled to a predetermined body part selected from a group consisting of: the hand of said surgeon, at least one of the fingers of the surgeon, the thigh of the surgeon, the neck of the surgeon, at least one of the legs of the surgeon, the knee of the surgeon, the head of the surgeon and any combination thereof.

In another preferred embodiment of the invention, each relevant surgical instrument is fitted at its maneuvering control end with a wireless transmitter (as part of the wearable operator) that transmits a unique code. While performing the surgical procedure, whenever the surgeon inserts a surgical instrument for the first time, he signals by activating the wearable operator so as to uniquely identify the surgical instrument.

According to one embodiment of the present invention, the wearable operator comprises an activating button, such that the activation of the same can be achieved by manually pressing the same.

According to another embodiment of the present invention, the wearable operator is activated manually or automatically.

According to one embodiment of the present invention, the activation of the wearable operator is achieved by means of depression on the same, voice activating the same, prolonged depression on the same, double clicking on the same and any combination thereof.

When the instrument is being inserted for the first time, the computer software identifies the instrument, analyzes the characteristics of the surgical instrument and keeps the characteristics in a database, thereby forming within the computerized system a unique signature for each of the instruments. Thereafter, upon activation of the wireless transmitter attached to each surgical instrument, the receiver receives the unique code, communicates it to the computer that identifies it with the signature stored at the insertion step and instructs the automated assistant to move the endoscope so as to achieve the desired focus.

In a further preferred embodiment, the selection is signified on the screen connected to the computer by displaying a graphic symbol upon the onscreen depiction of the surgical instrument.

In a further preferred embodiment the selection is confirmed by an additional mode of depression on the wireless transmitter, such as a prolonged depression of the wearable operator, authorizing the computer to instruct the automated assistant to change the view provided by the endoscope.

The device of the present invention has many technological advantages, among them:
- Simplifying the communication interface between surgeon and mechanical assistants.
- Seamless interaction with conventional computerized automated endoscope systems.
- Simplicity of construction and reliability.
- User-friendliness

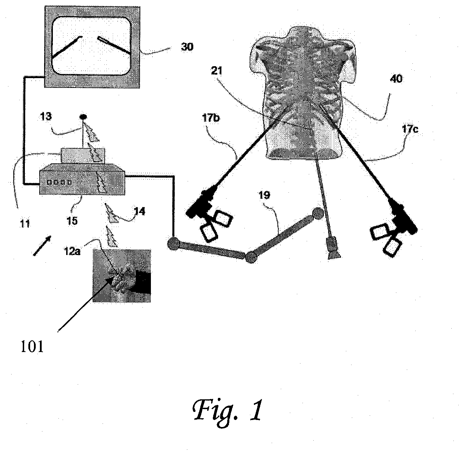

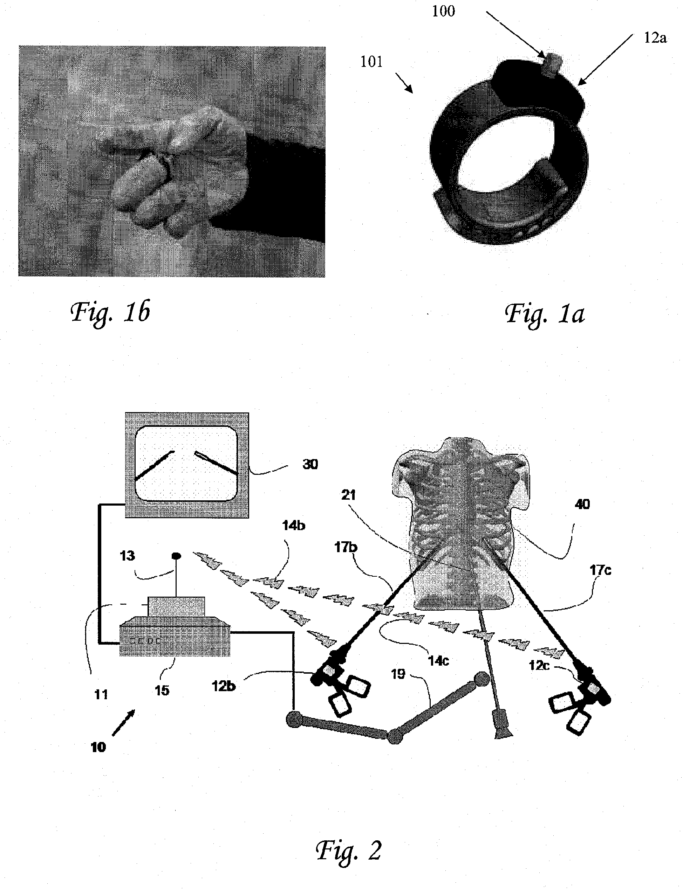

Reference is made now to Fig. 1, which is a general schematic view of an enhanced interface laparoscopic system comprising one or more wearable operators 101 (each of which comprises wireless transmitters 12a), that is worn by the surgeon (e.g., integrated within a bracelet or a ring).

Once the same is activated (e.g., depressed), it wirelessly transmits a single code wave 14 through aerial 13 to connected receiver 11 that produces a signal processed by computer 15, thereby assigning a particular code to one of two or more surgical instruments 17b and 17c within the patient 40 as the focus of the surgeon's attention.

Reference is now made to Figs. 1a-1b which illustrate a preferred embodiment of the wearable operator of the present invention.

According to this embodiment, the wearable operator is configured as a ring to be worn on the surgeon's finger (see Fig. 1b).

According to this embodiment, the wearable operator comprises a pressing key 100 (also referred to as pressing button 101d). Once the surgeon wishes to re-orient the endoscope so as to focus on the desired instrument (linked to said wearable operator), the surgeon presses the same.

Fig. 1a illustrates the wearable operator 101, in its ring-like configuration.

Fig. 1b illustrates the wearable operator 101, as worn by the surgeon.

According to another embodiment or the present invention, the wearable actuator may be attached to the maneuvering end of surgical instruments 17b and 17c.

It is appreciated that each surgical instrument has particular dimensions. Therefore, since there isn't a 'universal' shape of surgical instruments, each surgical instrument should be provided with a dedicated wearable operator. Thus, according to one embodiment of the present invention, a dedicated wearable operator is provided for each instrument.

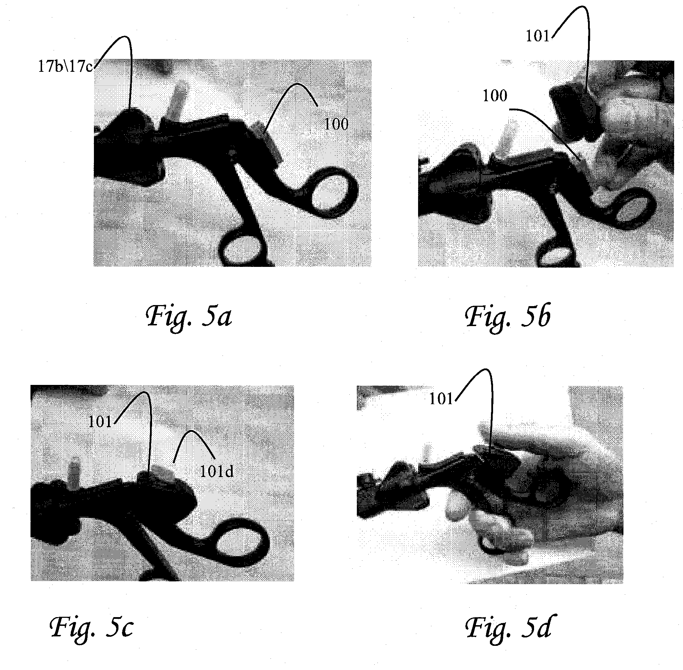

According to another embodiment of the present invention, a universal adaptor to be attached to any surgical instrument is provided (see further detail with respect to Figs. 5a-5e).

Once the wearable operator is operated, a conventional automated endoscope 21 is maneuvered by means of conventional automated arm 19 according to conventional computational spatial placement software contained in computer 15

Reference is made now to Fig. 2, which is a general schematic view of an enhanced interface laparoscopic system comprising one or more wearable operators (not shown in the figure). According to this embodiment, the wearable operators are worn on the surgical instrument. As described above, eEach of said wearable operators comprises a wireless transmitter (12b and 12c).

Each of the wireless transmitters 12b and 12c is attached, respectively, to the maneuvering means at the end of surgical instruments 17b and 17c. Once the wearable operator is activated (e.g., depressed), each transmits a unique code wave 14b and 14c through aerial 13 to connected receiver 11 that produces a signal processed by computer 15, thereby assigning a particular one of two or more surgical instruments 17b and 17c as the focus of the surgeon's attention. Accordingly, a conventional automated endoscope 21 is maneuvered by means of conventional automated arm 19 according to conventional computational spatial placement software contained in computer 15.

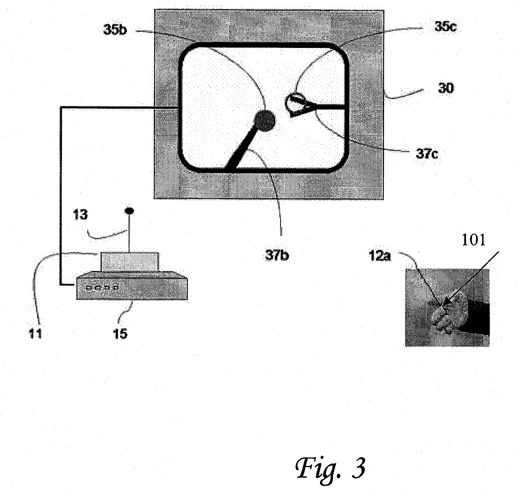

Reference is made now to Fig. 3, which is a schematic view of a method by which choice of instrumentation focus is achieved with a single wireless signal code, by means of video representations 35b and 35c of the actual surgical instruments (not represented in Fig. 3), displayed on a video screen as graphic symbols.

In this embodiment, on activation of the wearable operator 101 (e.g., by a light depression of the button on the wearable operator), wireless transmitter 12a emits a generic code that is received by receiver aerial 13 and communicated through connected receiver 11 to computer 15. Computer 15 shifts the graphically displayed symbol of choice 35b on video screen 30 from instrument to instrument until the required instrument is reached.

In this example, the wearable operator 101 is shaped as a ring and is worn on the surgeon's finger.

A prolonged depression of the wearable operator 101 confirms the selection, thereby signaling computer 15 to instruct the automated mechanical assistant to move the endoscope and achieve a camera view of the instrument area on screen 30.

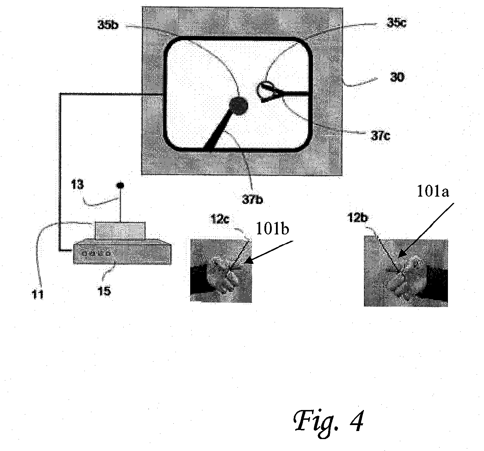

Reference is made now to Fig. 4, which is a schematic view of a method in which choice of instrumentation focus is achieved in the case where there are multiple wireless signal codes, by means of video representations 35b and 35c of the actual surgical instruments (not represented in Fig. 4), displayed as graphic symbols.

When the wearable operators 101a and 101b (and the wireless transmitters 12b and 12c, respectively) are being pressed, the same emit a signal which eventually results in the display or screen 30 of graphic symbol 35b on respective video representation 37b or, alternatively, of graphic symbol 35c on video representation 37c.

Confirmation of the selection may be achieved by prolonged depression of a button located on the wearable operator. Thus, a prolonged depression of the button on the wearable operator confirms the selection, thereby signaling computer 15 to instruct the automated mechanical assistant (not represented in Fig. 4) to move the endoscope (not represented in Fig. 4) and achieve a camera view of the instrument area on screen 30.

In another embodiment of this invention, when a prolonged depression of the buttons on the wearable operator confirms the selection, the computer software analyzes the characteristics of the surgical instrument and stores it in a database, thereby forming, within the computerized system, a database used for matching between each transmitting code and its associated surgical instrument.

From now on, when the surgeon presses again on this button, the receiver that receives the transmitted code communicates it to the computer software that identifies the code as a "known" code, matches it to the known parameters that were stored earlier in the database of surgical tools, and extracts the position of the tip of the surgical tool. When the position of the tool tip is known, the tracking software instructs the automated assistant to move the endoscope so as to achieve the desired focus.

In another embodiment of this invention, when the wearable operator is activated and an instrument is selected, the computer software analyzes the characteristics of the surgical instrument and stores it in a database, thereby forming, within the computerized system, a database used for matching between each transmitting code and a surgical instrument.

From now on, when the surgeon activates the wearable activator, the receiver that receives the transmitted code communicates it to the computer software that identifies the code as a "known" code and matches it to the known parameters that were stored earlier in database of the surgical tools, and extracts the position of the tip of the surgical tool. When the position of the tool tip is known, the tracking software instructs the automated assistant to move the endoscope so as to achieve the desired focus.

Reference is now made to Figs. 5a-5e illustrating another embodiment of the present disclosure.

As mentioned above, the wearable actuator may be attached to the maneuvering end of surgical instruments 17b and 17c. However, since each surgical instrument has particular dimensions, there is no 'universal' actuator that will fit every instrument. Thus, one should provide each of surgical instruments with a dedicated operator.

The present invention provides a universal adaptor 100 to be attached to the surgical instrument so as to overcome this disadvantage. The surgeon is able to couple the wearable operator 101 to the adaptor.

Reference is now made to Fig. 5a which illustrates the surgical instrument 17b or 17c to which the adaptor 100 is being attached.

Reference is now made to Fig. 5b which illustrates the coupling of the wearable operator 101 to the universal adaptor 100.

Reference is now made to Fig. 5c which illustrates the wearable operator 101 coupled to the adaptor and thus to the surgical instrument.

As mentioned above, according to one embodiment of the present invention, the wearable operator 101 comprises an activating button 101d (see Fig. 5c). Reference is now made to Fig. 5d which illustrates the activation of wearable operator 101. In Fig. 5d, activation is achieved by pressing on button 101d in wearable operator 101.

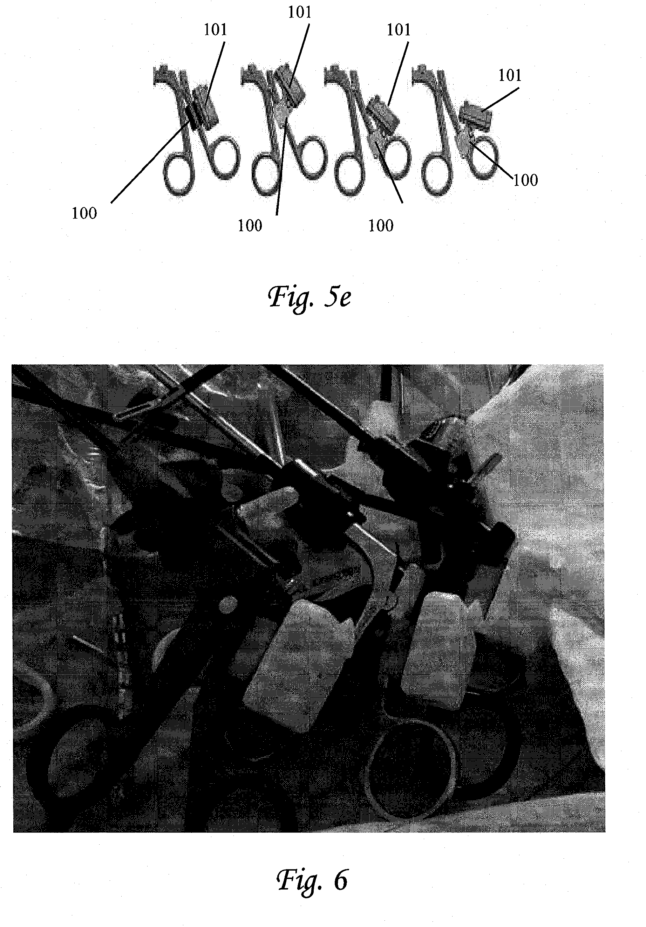

Figure 5e illustrates different positions for the wearable operator 101 (and the adaptor 100) on the surgical instrument.

In order to realize a position and range system, many well-known technologies may be used. For example, if the switches emit wireless signals then an array of antennas may be used to compare the power of the signal received at each antenna in order to determine the angle of the switch and the approximate range (distance and angle) between it and the camera holder mechanism. If the switch emits ultrasound (US), then US microphones can be used to triangulate the position of the switch. The same can be done for light emitting switches.

Reference is now made to Fig. 6 which illustrates the adjustability of the wearable operator 101. As can be seen from the figure, the wearable operator 101 can be fitted to a variety of different tools, each of which is characterized by a different size and shape.

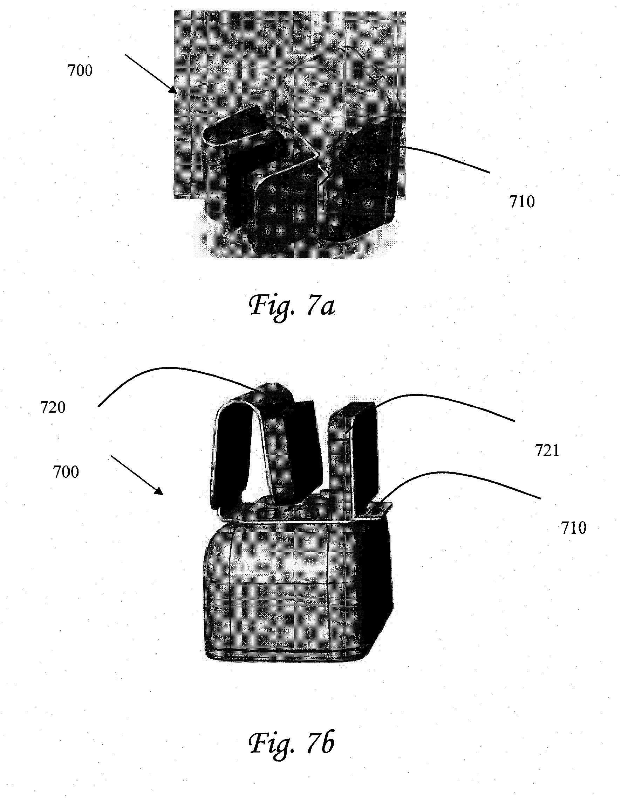

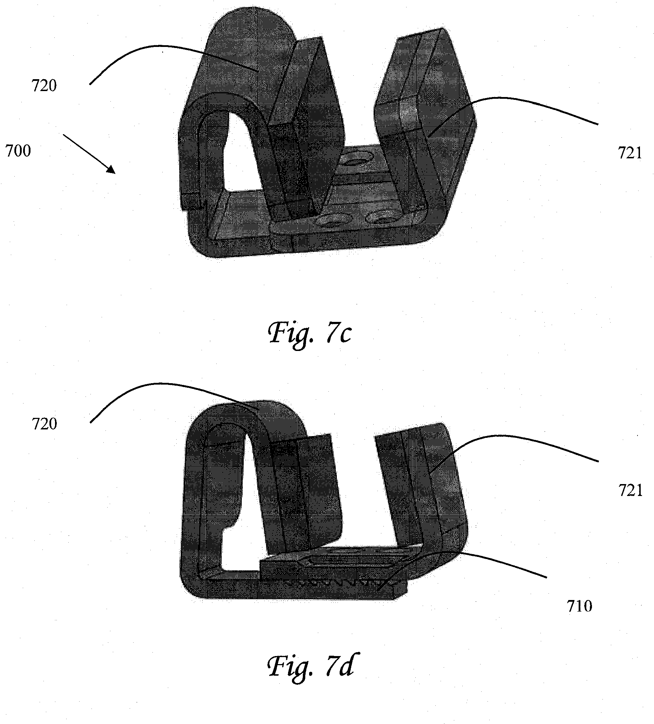

Reference is now made to Figs. 7a-7d illustrating one embodiment of the wearable operator 700 and the adjustable means by which it may be attached to a surgical tool.

According to this embodiment, the wearable operator 700 comprises a unidirectional coupling (e.g., ratchet 710).

Once the wearable operator 700 is secured to the surgical tool, the wearable operator 700 is adjusted to the size and dimensions of the surgical tool by means of a unidirectional catch(e.g., ratchet 710).

According to another embodiment, the wearable operator 700 comprises a body having at least two portions 720 and 721 (see Fig. 7b). Said portions are adapted to 'grasp' the surgical tool such that when the wearable operator 700 is coupled to the surgical tool, fine-tuned movement of the two body portions is obtainable so as to provide said tight-fit coupling between said two portions and said instrument.

According to another embodiment, one of the two portions (either 720 or 721) is rotationally movable relative to the other, such that when said wearable operator is coupled to said instrument, fine-tuned movement of said two body portions is obtainable so as to provide said tight-fit coupling between said two portions and said instrument.

According to another embodiment, the two portions (721 and 720) are rotationally movable relative to each other, such that when the wearable operator is coupled to said instrument, fine-tuned movement of said two body portions is obtainable so as to provide said tight-fit coupling between said two portions and said instrument.

The movement of either portion 720 or portion 721 relative to the other is obtained by fixating the position of either portion 720 or portion 721 and coupling the other portion to e.g., a unidirectional catch (e.g., ratchet) 710 or a two-way directional catch 710 on the body of the wearable operator.

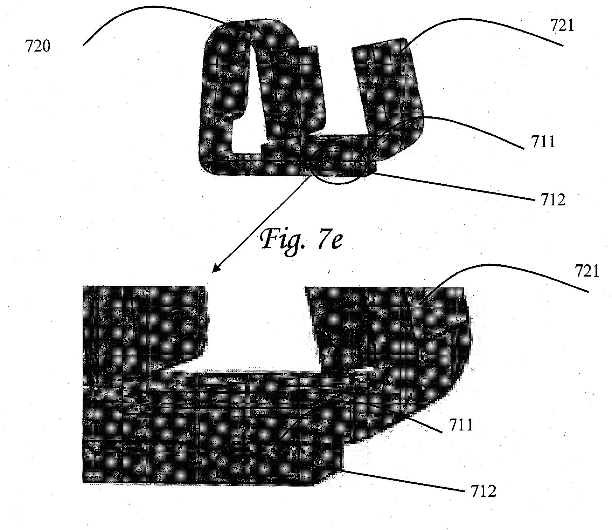

According to another embodiment, the movement of either portion 720 or portion 721 relative to the other is obtained by providing one portion, e.g., portion 721 with cog-like teeth 711 and the body of the wearable operator with cog-like teeth 712 matching with cog-like teeth 711 (see Fig. 7e). In such a way portion 721 can be linearly moved relative to portion 720.

According to another embodiment of the present invention, the wearable operator is a ring to be worn on the physician's hand.

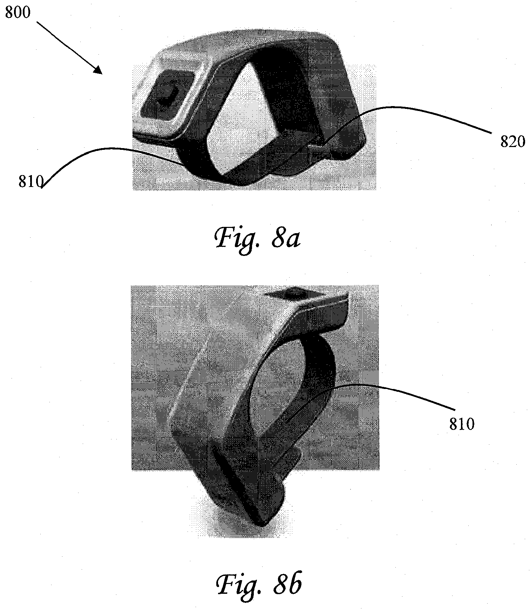

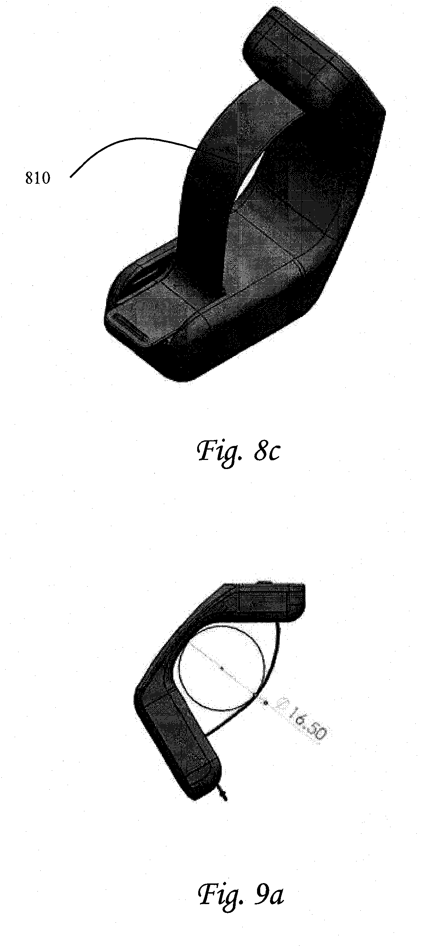

Reference is now made to Figs. 8a-8c, illustrating another embodiment of the present disclosure, which provides the best adjustment of the wearable operator 800 to the operator's hand.

According to another embodiment, the wearable operator 800 is adjustable by means of flexible and stretchable silicone and/or rubber strip 810 and a loop-closing means. The loop-closing means is adapted to close a loop with the flexible and stretchable strip. Together, the flexible and stretchable strip and the loop-closing means are provided so as to fit the wearable operator to at least one selected from a group consisting of (a) said predetermined location of said different instruments; (b) said predetermined body part of said surgeon, each of which is characterized by a different size and shape.

As will be disclosed hereinafter, the loop-closing means 820 can be e.g., a unidirectional catch, a rack, a peg or any other mechanism known in the art.

According to another embodiment, the silicone and/or rubber strip 810 is passed through a unidirectional catch (e.g., ratchet 820), such that, when the physician wears the wearable operator 800, he adjusts the same by pulling the silicone and/or rubber strip 810 through the ratchet 820.

According to another embodiment, the silicone and/or rubber strip 810 is rotated around rack or peg 820 such that, when the physician wears the wearable operator 800, he adjusts the same by pulling the silicone and/or rubber strip 810 around the peg 820.

According to this embodiment, the silicone and/or rubber strip 810 is characterized by a varied width along its length. More specifically, at least a portion of the silicone and/or rubber strip 810 is characterized by a greater width, such that when the same is twisted/rotated around peg 820 and reaches the wider portion, the same is fixedly secured to the wearable operator 800.

According to another embodiment, the silicone and/or rubber strip 810 is characterized by different surface roughnesses along its length. More specifically, at least a portion of the silicone and/or rubber strip 810 is characterized by e.g., an abrasive or rough surface such that when the same is twisted/rotated around peg 820 and reaches the rougher portion, the same is fixedly secured to the wearable operator 800.

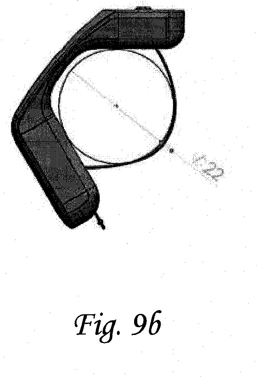

Reference is now made to Figs. 9a-9b illustrating the 'adjustability' of the wearable operator. As can be seen, the wearable operator can be fit to and be secured to both 'wider' fingers (see fig. 9b) and 'narrower' fingers (see fig. 9a).

It is appreciated that certain features of the invention which are, for clarity, described in the context of separate embodiments, can also be provided in combination in a single embodiment. Conversely, various features of the invention which are, for brevity, described in the context of a single embodiment, can also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims. In addition, citation or identification of any reference in this application shall not be construed as an admission that such reference is available as prior art to the present invention.

## Claims

1. An enhanced interface laparoscopy device, comprising:
a. at least one endoscope (21), mechanically interconnected to an automated assistant; said automated assistant is adapted to maneuver said endoscope (21) to a desired location;
b. at least one instrument (17);
c. at least one operator (700) comprising at least one wireless transmitter (12), adapted to transmit a signal once said at least one operator is activated; said at least one operator is either wire or wirelessly in communication with said at least one of said instrument (17);
d. at least one wireless receiver (11); adapted to receive said signal sent by said transmitter (12);
e. at least one laparoscopy computerized system, in communication with said wireless receiver (11), adapted to provide a visual onscreen depiction of said at least one instrument (17) to be selected following the activation of said at least one operator; and,
f. at least one video screen (30);
wherein said device is adapted to control and to direct said endoscope (21) via said laparoscopy computerized system and said automated assistant on said instrument to be selected following the activation of said at least one operator (700);
**characterized in that** said at least one operator (700) is wearable, and wherein said wearable operator (700) comprises a body having two portions (720, 721) partially overlapping each other; said two portions are adapted to grasp and hold said instrument (17) there-between, such that a tight-fit coupling between said two portions and said instrument (17) is obtained,
wherein one of the two portions (720, 721) is provided with a unidirectional or a two-way directional catch (710), such that the one of the two portions (720, 721) is adapted to be linearly moved relative to the other of the two portions (720, 721).

2. The device according to claim 1, wherein said communication between said at least one of said wearable operators (700) and said instrument (17) is either wire or wirelessly coupling.

3. The device according to claim 1, wherein said wearable operator (700) is worn by said surgeon on a predetermined body part; wherein said predetermined body part is selected from a group consisting of: the hand of said surgeon, at least one of the fingers of said surgeon, and any combination thereof.

4. The device according to claim 1, wherein at least one of the following is being held true (a) said wireless transmitter (12) is (i) freestanding; or, (ii) fitted to each of said at least one instruments (17); (b) said wireless transmitter (12) is adapted to locate the position of at least one of said instruments (17); (c) selection of said at least one instrument (17) is obtained by activating said at least one wearable operator (700), wherein the activation of said at least one wearable operator (700) is obtained by depression on a predetermined location in said wearable operator (700), prolonged depression on the predetermined location, double clicking on the predetermined location and any combination thereof; (d) said laparoscopy computerized system comprises at least one surgical instrument spatial location software, adapted to locate the 3D spatial position of said at least one instrument and said laparoscopy computerized system further comprises at least one automated assistant maneuvering system, said automated assistant maneuvering system is coupled to said endoscope (21) and is adapted to direct said endoscope (21) to said at least one instrument, said instrument selected following the activation of said at least one wearable operator (700).

## Patentansprüche

1. Ein Laparoskopiegerät mit verbesserter Schnittstelle, bestehend aus:
a. mindestens einem Endoskop (21), das mechanisch mit einem automatisierten Assistenten verbunden ist. Der automatisierte Assistent ist so ausgelegt, dass er das Endoskop (21) an eine gewünschte Stelle manövrieren kann;
b. mindestens ein Instrument (17);
c. mindestens eine Operatorvorrichtung (700), der mindestens einen drahtlosen Sender (12) umfasst, der dazu ausgelegt ist, ein Signal zu senden, sobald der mindestens eine Operatorvorrichtung aktiviert ist. Die mindestens eine Operatorvorrichtung steht entweder kabelgebunden oder kabellos in Kommunikation mit dem mindestens einen der Instrumente (17);
d. mindestens einen drahtlosen Empfänger (11); der zum Empfang des von dem Sender (12) gesendeten Signals geeignet ist;
e. mindestens einem computergestützten Laparoskopiesystem, das mit dem drahtlosen Empfänger (11) in Verbindung steht und dazu ausgelegt ist, eine visuelle Darstellung des mindestens einen Instruments (17) auf dem Bildschirm zu liefern, das nach der Aktivierung der mindestens einen Operatorvorrichtung auszuwählen ist; und,
f. mindestens einen Videobildschirm (30);
wobei die Vorrichtung dazu geeignet ist, das Endoskop (21) über das computerisierte Laparoskopiesystem und den automatischen Assistenten an dem Instrument zu steuern und zu lenken, das nach der Aktivierung der mindestens einen Operatorvorrichtung (700) ausgewählt werden soll;
**dadurch gekennzeichnet, dass** die mindestens eine Operatorvorrichtung (700) tragbar ist, und wobei die tragbare Operatorvorrichtung (700) einen Körper mit zwei Abschnitten (720, 721) aufweist, die einander teilweise überlappen; wobei die beiden Abschnitte so ausgelegt sind, dass sie das Instrument (17) dazwischen greifen und halten, so dass eine eng anliegende Verbindung zwischen den beiden Abschnitten und dem Instrument (17) entsteht,
wobei einer der beiden Abschnitte (720, 721) mit einer unidirektionalen oder einer bidirektionalen Sperre (710) versehen ist, so dass der eine der beiden Abschnitte (720, 721) so angepasst ist, dass er relativ zum anderen der beiden Abschnitte (720, 721) linear bewegt werden kann.

2. Vorrichtung nach Anspruch 1, wobei die Kommunikation zwischen der mindestens einen der tragbaren Operatorvorrichtungen (700) und dem Instrument (17) entweder kabelgebunden oder kabellos erfolgt.

3. Vorrichtung nach Anspruch 1, wobei die tragbare Operatorvorrichtung (700) vom Chirurgen an einem vorbestimmten Körperteil getragen wird; wobei der vorbestimmte Körperteil aus einer Gruppe ausgewählt wird, die aus der Hand des Chirurgen, mindestens einem der Finger des Chirurgen und einer beliebigen Kombination davon besteht.

4. Vorrichtung nach Anspruch 1, bei der mindestens eines der folgenden gilt: (a) der drahtlose Sender (12) ist (i) freistehend; oder (ii) an jedem der mindestens einen Instrumente (17) angebracht; (b) der drahtlose Sender (12) ist geeignet, die Position mindestens eines der Instrumente (17) zu lokalisieren; (c) die Auswahl des mindestens einen Instruments (17) erfolgt durch Aktivierung der mindestens einen tragbaren Operatorvorrichtung (700),
wobei die Aktivierung der mindestens einen tragbaren Operatorvorrichtung (700) durch Eintauchen in eine vorbestimmte Stelle in der tragbaren Operatorvorrichtung (700), längeres Eintauchen in die vorbestimmte Stelle, Doppelklicken auf die vorbestimmte Stelle und eine beliebige Kombination davon erfolgt; (d) das computergestützte Laparoskopiesystem mindestens eine Software zur räumlichen Lokalisierung von chirurgischen Instrumenten umfasst, die geeignet ist, die räumliche 3D-Position des mindestens einen Instruments zu lokalisieren, und das computergestützte Laparoskopiesystem ferner mindestens ein automatisiertes Assistentenmanöversystem umfasst; das automatisierte Assistentenmanöversystem ist mit dem Endoskop (21) gekoppelt und geeignet, das Endoskop (21) auf das mindestens eine Instrument zu richten, wobei das Instrument nach der Aktivierung der mindestens einen tragbaren Operatorvorrichtung (700) ausgewählt wird.

## Revendications

1. Dispositif amélioré de laparoscopie à interface, comprenant :
a. au moins un endoscope (21) interconnecté par voie mécanique à un assistant automatisé, ledit assistant automatisé étant conçu pour manœuvrer ledit endoscope (21) jusqu'à un endroit désiré ;
b. au moins un instrument (17) ;
c. au moins un opérateur (700) comprenant au moins un émetteur-récepteur sans fil (12) conçu pour la transmission d'un signal une fois que ledit au moins un opérateur est activé, ledit au moins un opérateur étant mis en communication soit par fil, soit sans fil avec au moins un dudit au moins un instrument (17) ;
d. au moins un récepteur sans fil (11), conçu pour la réception dudit signal envoyé par ledit émetteur-récepteur (12) ;
e. au moins un système informatisé de laparoscopie, mis en communication avec ledit récepteur sans fil (11), conçu pour procurer une représentation visuelle sur écran dudit au moins un instrument (17) qui doit être sélectionné suite à l'activation d'au moins un dudit au moins un opérateur ; et
f. au moins un écran vidéo (30) ;
dans lequel ledit dispositif est conçu pour la commande et pour le guidage dudit endoscope (21) par l'intermédiaire dudit système informatisé de laparoscopie et dudit assistant automatisé sur ledit instrument qui doit être sélectionné suite à l'activation dudit au moins un opérateur (700) ;
**caractérisé en ce que** ledit au moins un opérateur (700) peut être porté ; et dans lequel ledit opérateur portable (700) comprend un corps possédant deux portions (720, 721) qui se recouvrent en partie l'une l'autre, lesdites deux portions étant conçues pour saisir et maintenir ledit instrument (17) entre elles d'une manière telle que l'on obtient un accouplement en ajustement serré entre lesdites deux portions et ledit instrument (17) ;
dans lequel une des deux portions (720, 721) est munie d'un élément de préhension directionnel de type unidirectionnel ou bidirectionnel (710), d'une manière telle que ladite une portion parmi les deux portions (720, 721) est conçue pour un déplacement linéaire par rapport à ladite autre portion parmi les deux portions (720, 721).

2. Dispositif selon la revendication 1, dans lequel ladite mise en communication entre ledit au moins un élément choisi parmi lesdits opérateurs portables (700) et lesdits instruments (17) est un accouplement par fil ou sans fil.

3. Dispositif selon la revendication 1, dans lequel ledit opérateur portable (700) est porté par ledit chirurgien sur une partie du corps prédéterminée ; dans lequel ladite partie du corps prédéterminée est choisie parmi un groupe constitué par la main dudit chirurgien, au moins un des doigts dudit chirurgien, ainsi que l'une quelconque de leurs combinaisons.

4. Dispositif selon la revendication 1, dans lequel au moins une des indications qui suivent est vraie : (a) ledit émetteur-récepteur sans fil (12) est (i) autoportant ou (ii) équipe chacun dudit au moins un instrument (17) ; (b) ledit émetteur-récepteur sans fil (12) est conçu pour la localisation de la position d'au moins un instrument parmi lesdits instruments (17) ; (c) la sélection dudit au moins un instrument (17) est obtenue par l'activation dudit au moins un opérateur portable (700) ; dans lequel l'activation dudit au moins un opérateur portable (700) est obtenue en exerçant une pression sur un endroit prédéterminé dans ledit opérateur portable (700), en appuyant de manière prolongée sur l'endroit prédéterminé, en double cliquant sur l'endroit prédéterminé, et par l'intermédiaire de l'une quelconque de leurs combinaisons ; (d) ledit système informatisé de laparoscopie comprend au moins un logiciel de localisation spatiale d'un instrument chirurgical, conçu pour la localisation de la position spatiale en trois dimensions dudit au moins un instrument, et ledit système informatisé de laparoscopie comprend en outre au moins un système destiné à manœuvrer l'assistant automatisé, ledit système destiné à manœuvrer l'assistant automatisé étant accouplé audit endoscope (21) et étant conçue pour diriger ledit endoscope (21) en direction dudit au moins un instrument, ledit instrument étant sélectionné suite à l'activation dudit au moins un opérateur portable (700).
